# EUROPEAN PATENT APPLICATION

(11) **EP 4 279 922 A1**
(43) Date of publication of application: **22.11.2023**
(21) Application number: 22739025.9
(22) Date of filing: 12.01.2022
(51) Int. Cl.: G01N 33/543, G01N 33/577, G01N 33/53

(54) **LATEX ENHANCED COMPETITIVE TURBIDIMETRIC IMMUNOASSAY DETECTION METHOD AND KIT**

(30) Priority: 18.01.2021 CN 202110064759
(71) Applicant: SHANGHAI YUNZE BIOTECHNOLOGY CO., LTD, Shanghai 201112 (CN); SHANGHAI GENEXT MEDICAL TECHNOLOGY CO., LTD, Shanghai 201114 (CN)
(72) Inventor: WU, Fengbo, Shanghai 201112 (CN); LIN, Weijing, Shanghai 201112 (CN); LIU, Binhu, Shanghai 201112 (CN)
(74) Representative: Isarpatent
(86) International application number: PCT/CN2022/071529
(87) International publication number: WO 2022/152147

(57) **Abstract**

A method for improving the sensitivity of latex enhanced competitive turbidimetric immunoassay analysis, and a detection kit based on the method. By using multiple labeling of an antibody with biotin and a binding reaction between biotin and avidin, a latex enhanced competitive turbidimetric immunoassay analysis method can be established with trace BLAAA, so as to remarkably improve detection sensitivity.

## Description

The present application claims the priority of Chinese patent application CN2021100647596, filed on January 18, 2021, the contents of which are incorporated herein by reference in its entirety.

### Technical field

The present invention relates to the field of biochemical detection, specifically relates to latex enhanced competitive turbidimetric immunoassay detection method and kit using the same.

### Background

Latex enhanced turbidimetric immunoassay detection (LETIA) is a classical immunoassay method, wherein an antibody or antigen is coupled to the surface of a nano-sized latex microsphere, and microsphere aggregates are formed by the reaction of the antibody or antigen on the surface of the latex microsphere with the analyte contained in a specimen, utilizing the highly specific binding between antibody and antigen; furthermore, the absorbance of the immune reaction system at a specific wavelength is changed, and the concentration of the analyte in the specimen is calculated by measuring this change. So far, LETIA has been widely used in clinical medicine, involving many fields of tumor, rheumatism, liver function, renal function and drug concentration monitoring. However, although LETIA has been widely used in routine clinical analysis, it is still a challenge to accurately quantify the extremely low concentration of analyte in a specimen with LETIA because of its low sensitivity.

Latex enhanced competitive turbidimetric immunoassay is an implementation of LETIA, which realizes the quantification of the analyte by competitively binding of a limited amount of anti-analyte antibody to the analyte contained in a specimen and the analyte in a detection system with a fixed amount. It is often used for the detection of small molecular analytes, such as digoxin, tacrolimus, cyclosporine, methotrexate, imatinib, aflatoxin, clenbuterol and Sudan red, etc.

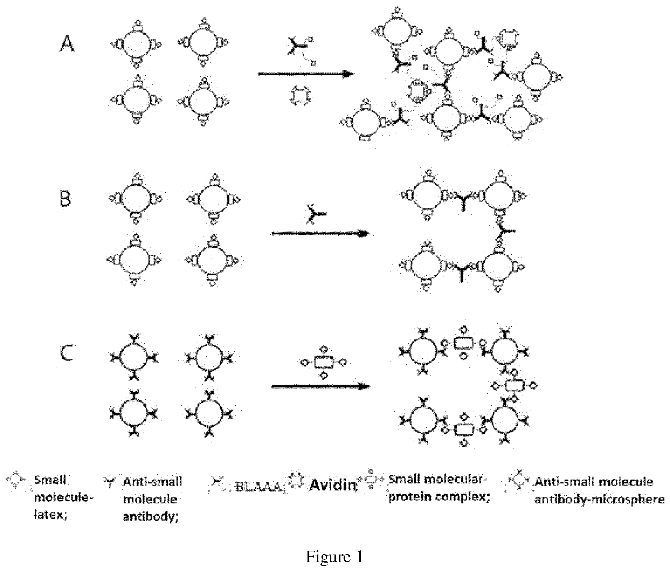

The enhanced competitive turbidimetric immunoassay, which is well known to those skilled in the art, usually adopts two modes: the direct mode with the small molecular analyte coupled to a microsphere and the direct mode with the antibody coupled to a microsphere, and their principles are shown in Figure 1-B and Figure 1-C respectively.

In the direct method mode with the small molecular analyte coupled to a microsphere (Figure 1-B), the small molecular analyte contained in the specimen binds to a limited amount of anti-analyte antibody competitively with the small molecular analyte coupled to the surface of the microsphere. When the specimen does not contain the analyte, the antibody can bridge two different microspheres through its two binding sites, resulting in agglutination. When the specimen contains the analyte, the binding sites of an antibody is occupied by the analyte, and the agglutination of latex microspheres is inhibited. In this mode, the part of antibodies whose two binding sites are both bound with two analyte molecules on the surface of the same latex microsphere will lose the ability to bridge two latex microspheres, and thus cannot cause agglutination. Despite sufficient process optimization, the maximum sensitivity of tacrolimus of tacrolimus LETIA established by the inventor with this model is only about 6.9ng/mL.

The direct mode with the antibody coupled to a microsphere (Figure 1-C) needs to firstly couple small molecules with proteins or other carriers to prepare a carrier-small molecule complex, so that a single carrier molecule contains multiple small molecules. Due to the multivalence of carrier-small molecule complex and antibody-latex microspheres, the carrier-small molecule complex and antibody-latex microspheres can easily cause agglutination under appropriate conditions, while the small molecule analyte in a specimen can inhibit this agglutination reaction by occupying the antibody binding site on the surface of latex microspheres. According to the degree of inhibition of agglutination reaction, the concentration of small molecule analyte in a specimen can be quantified. Using this model, the inventors developed a human whole blood tacrolimus-latex enhanced competitive turbidimetric immunoassay detection reagent (CFDA registration number: 20192400305). Although it has excellent accuracy, the limit of detection (LOD) of tacrolimus obtained is only about 1.0ng/mL. 1.0ng/mL of LOD is sensitive enough for most clinical monitoring of tacrolimus. However, for organ transplant patients who take combined immunosuppressive drugs (A comparative, randomized trial of concentration-controlled sirolimus combined with reduced-dose tacrolimus or standard-dose tacrolimus in renal allograft recipients Transplant proceedings, 2013, 45, 2133-2140) and some patients with autoimmune diseases treated with low-dose tacrolimus (Changes in the blood level, efficacy, and safety of tacrolimus in pregnancy and the lactation period in patients with systemic lupus erythematous. Lupus. 2018,27:2245-2252), further improvement of detection sensitivity will help to detect drugs with low-concentration more accurately in the blood of these patients.

Therefore, finding a method that can effectively improve the sensitivity of latex enhanced competitive turbidimetric immunoassay will help to detect the concentration of small molecule analyte with low-content more accurately in a specimen more accurately, and expand the range of analyte in the latex enhanced competitive turbidimetric immunoassay.

### Summary of the invention

In order to solve the technical problems that existing latex enhanced competitive turbidimetric immunoassay has low detection sensitivity to accurately detect the small molecule analyte with low concentration, the present invention provides a new detection mode of latex enhanced competitive turbidimetric immunoassay (as shown in A in Figure 1) and the kit thereof. In this mode, due to the multiple labeling of biotin to anti-analyte antibodies, the binding of biotin labeled anti-analyte antibodies (BLAAAs) to the analyte on the surface of the latex microspheres can introduce a large amount of biotin onto the surface of the latex microspheres. Even if a small amount of BLAAA is used, an effective agglutination reaction can be initiated in the presence of tetravalent avidin. Therefore, this mode can establish a latex enhanced competitive turbidimetric immunoassay method with a small amount of BLAAA, which significantly improves the detection sensitivity.

The present invention provides a method for latex enhanced competitive turbidimetric immunoassay, comprising the following steps:
(1) obtaining a mixed solution 1 by mixing and incubating a biotin labeled anti-analyte antibody (BLAAA) with a calibrator or specimen;
(2) mixing the mixed solution 1 in step (1) with avidin and a latex microsphere coupled with the analyte, and detecting initial absorbance A1, detecting absorbance A2 after incubation, and calculating absorbance increment ΔA, ΔA =A2-A1;
(3) establishing a calibration curve for the concentration of the analyte comprised in the calibrator by the absorbance increment ΔA of the calibrator, and realizing the quantitative detection of the concentration of the analyte in the specimen by comparison of the absorbance increment ΔA of the specimen;
or,
(1) obtaining mixed solution 1 by mixing and incubating a biotin labeled anti-analyte antibody (BLAAA) with a calibrator or specimen;
(2) obtaining mixed solution 2 by mixing and incubating the mixed solution 1 in step (1) with the latex microspheres coupled with the analyte;
(3) mixing the mixed solution 2 in step (2) with avidin, and detecting the initial absorbance A1, detecting the absorbance A2 after incubation, and calculating the absorbance increment ΔA, ΔA =A2-A 1;
(4) establishing a calibration curve for the concentration of the analyte contained in the calibrator by the absorbance increment ΔA of the calibrator, and realizing the quantitative detection of the concentration of the analyte in the specimen by comparison of the absorbance increment ΔA of the specimen.

In the present invention, the anti-analyte antibody refers to a protein which can generate specific immune binding reaction with an analyte and has the structural characteristics of an antibody.

In the present invention, the analyte refers to various small molecular substances contained in human body, animal body, plant body and abiotic body in nature that can be detected by latex enhanced competitive turbidimetric immunoassay, such as: digoxin, Tacrolimus, cyclosporine, clozapine, aripiprazole, olanzapine, diazepam, quetiapine, risperidone, amitriptyline, duloxetine, nortriptyline, venlafaxine, citalopram, imipramine, sertraline, carbamazepine, oxcarbazepine, valproic acid, phenytoin, lamotrigine, levetiracetam, topiramate, aflatoxin, amphotericin B, voriconazole, posaconazole, itraconazole, teicoplanin, vancomycin, linezolid, amikacin, polymyxin B, tigecycline, chloramphenicol, melamine, fumonisin, aflatoxin M1, aflatoxin B1, clenbuterol or salbutamol.

In the present invention, the biotin labeled anti-analyte antibody can be prepared by a biotin labeling reagent with a biotin labeling method commonly used in the art. The biotin labeling reagent includes, but is not limited to, biotin-succinimide esters with various arm lengths that react with the amino group of a protein, biotin-maleimide derivatives with various arm lengths that react with the sulfhydryl group of a protein, and biotin-amino derivatives with various arm lengths that react with the carboxyl group of a protein. Those skilled in the art can determine the labeling rate of biotin to the anti-analyte antibody by HABA-Avidin method with a commercial kit (e.g. Biotin Quantitation Kit; Thermo-fisher, US).

The molar ratio of biotin to the anti-analyte antibody in the BLAAA is preferably 5-25:1, more preferably 10-20:1, and most preferably 12-17:1.

The BLAAA is stored and used in the form of pH buffer. The concentration of BLAAA is generally 1-1000 ng/mL, preferably 5-200 ng/mL.

In the present invention, the avidin refers to a group of proteins that contain four biotin binding sites and can bind to the biotin molecule with high affinity, such as avidin, neutravidin, streptavidin or polymers thereof. The avidin is stored and used in the form of pH buffer, and the concentration of the avidin is preferably 0.5-500 µg/mL, more preferably 2-100 µg/mL.

In the present invention, the latex microsphere coupled with the analyte can be prepared by conventional methods in the art, such as chemical coupling or physical adsorption. If the analyte contains an active functional group that can react directly with the surface group of a latex microsphere as coupling reaction, a corresponding chemical method can be selected to implement coupling according to the functional groups contained on the latex microsphere and the analyte. Alternatively, the analyte can be coupled to a protein to obtain a protein-analyte complex, and then the protein-analyte complex is coupled to the surface of the latex microsphere through chemical coupling between the reactive functional groups contained in the protein and the surface functional groups of the latex microsphere. Thus, the indirect coupling of the analyte to the latex microsphere is completed.

In the latex microsphere coupled with the analyte, the latex microsphere can be selected from latex microspheres commonly used in the art, for example, a polystyrene latex microsphere with carboxyl on the surface. The average particle size of the polystyrene latex microsphere with carboxyl on the surface is preferably 50-500 nm, more preferably 100-400 nm. The carboxyl density on the surface of the polystyrene latex microsphere with carboxyl on the surface is preferably 0.02-0.40 mmol/g, more preferably 0.06-0.20 mmol/g.

The latex microsphere coupled with the analyte is used in the form of pH buffer. The concentration of the latex microsphere coupled with the analyte is generally 0.01%-1%, preferably 0.05%-0.5%.

In the present invention, the buffering agent in the pH buffer can be a buffering pair composed of any one of the following reagents and its conjugated acid or its conjugated base according to the convention in the art: one or more of Trihydroxymethyl aminomethane (Tris), N-2-hydroxyethyl piperazine-N-3 propanesulfonic acid (HEPPS), 3-(N-Morphine) propane sulfonic acid (MOPS), triethanolamine (TRA), diethanolamine (DEA), 2-methyl-2-amino-1 propanol (AMP), N-bis(2-hydroxyethyl)-2-aminoethanesulfonic acid (BESN), (cyclohexylamine)-1-propanesulfonic acid (CAPS), (cyclohexylamine)-1-ethanesulfonic acid (CHES), (N-morphine) ethanesulfonic acid (MES), Piperazin-N, N-bis (2-ethanesulfonic acid) (PIPES), Piperazin-N, N-bis (2-hydroxyethanesulfonic acid) (POPSO), Tris (hydroxymethyl) methyl-3-aminopropanesulfonic acid (TAPSN) and tris (hydroxymethyl) methyl-2-aminosulfonic acid (TESN). The pH of the pH buffer is preferably 4.5-9.5, more preferably 6.0-8.0. The concentration of the buffering agent can be conventionally selected, and generally it is preferably 10-500 mM.

Adding salt compounds to the latex microsphere coupled with the analyte or the pH buffer containing the BLAAA can increase the ionic strength of the solution, which is conducive to the stable storage of the microsphere and the BLAAA. The ionic strength mainly comes from alkali metal salt solution, including but not limited to NaCl, KCl, Na₂HPO₄, NaH₂PO₄, K₂HPO₄, KH₂PO₄, Na₂CO₃, NaHCO₃ and K₂CO₃. The concentration of the alkali metal salt is preferably 0.01-2.00 mol/L, more preferably 0.05-1.0 mol/L.

The pH buffer may also contain one or more of coagulants, preservatives, surfactants, and sealants.

Here, the coagulant can accelerate the reaction between the analyte and the anti-analyte antibodies, as well as the reaction between avidin and biotin. The type and concentration of the coagulant can be selected conventionally in the art. In the present invention, the coagulant is preferably polyethylene glycol (PEG) with a molecular weight greater than 3000 Daltons, and more preferably PEG with a molecular weight between 6000 and 30000 Daltons. The concentration of the coagulant is generally 0.1-10 wt%.

Here, the preservative can be selected conventionally in the art, such as selected from Proclin300, sodium azide, Kathon, and gentamicin.

Here, the surfactant can be selected conventionally in the art. In the invention, the preferred nonionic surfactant includes but is not limited to dehydrated sorbitol polyoxyethylene ether laurate surfactant, dehydrated sorbitol polyoxyethylene ether oleate surfactant and octylphenol polyoxyethylene ether surfactant. The concentration of the surfactant is generally 0.01-1 wt%.

Here, the sealant can be can be selected conventionally in the art, such as Blockmaster DB1130 and bovine serum albumin (BSA).

The present invention also provides a kit for latex enhanced competitive turbidimetric immunoassay, comprising:
(1) reagent 1 (R1), wherein the R1 is a pH buffer solution comprising BLAAA, and the BLAAA is a biotin labeled anti-analyte antibody;
(2) reagent 2(R2), wherein the R2 is a pH buffer solution comprising avidin and a latex microsphere coupled with the analyte;
(3) a calibrator.
Or,
(1) reagent 1 (R1), wherein the R1 is a pH buffer solution comprising BLAAA, the BLAAA is a biotin labeled anti-analyte antibody;
(2) reagent 2 (R2), wherein the R2 is a pH buffer solution comprising a latex microsphere coupled with the analyte;
(3) reagent 3 (R3), wherein the R3 is a pH buffer solution comprising avidin;
(4) a calibrator.

The calibrator is a standard solution or a freeze-dried product comprising the analyte with a known concentration, being used for establishing a calibration curve to realize the quantitative detection of the analyte.

To simplify the composition of the kit and facilitate its use, the kit for latex enhanced competitive turbidimetric immunoassay preferably only comprises: R1 comprising BLAAA, R2 comprising avidin and a latex microsphere coupled with the analyte, and a calibrator comprising the analyte with a known concentration.

Preferably, the components of the kit for latex enhanced competitive turbidimetric immunoassay are as described above.

In all technical solutions of the present invention, unless otherwise specified, the concentration refers to the concentration before use (before mixing), and the percentage (%) is the mass/volume ratio (g/100mL). On the basis of complying with common knowledge in the art, the above-mentioned preferred conditions can be arbitrarily combined to obtain the preferred examples of the present invention.

In all technical solutions of the invention, unless otherwise specified, reagents and raw materials used in the present invention are commercially available.

The positive effect of the present invention is that the method and the kit for latex enhanced competitive turbidimetric immunoassay provided by the present invention can significantly improve the detection sensitivity of the small molecule analyte. Based on the improvement of detection sensitivity, this method can be used to accurately detect the concentration of a small molecule analyte with low content in a specimen, which overcomes the limitation of low detection sensitivity of latex enhanced competitive turbidimetric immunoassay to a certain extent, so that more analytes with low concentration can be accurately detected to expand the range of analytes to be detected for latex enhanced competitive turbidimetric immunoassay.

### Brief description of the drawings

Figure 1 shows three modes of latex enhanced competitive turbidimetric immunoassay; wherein, A in Figure 1 is the mode of latex enhanced competitive turbidimetric immunoassay proposed by the present invention, B in Figure 1 is the direct method of analyte coupled with microsphere, and C in Figure 1 is the direct method of antibody coupled with microsphere.
Figure 2 shows the agglutination progress curves of six calibrators when the dosage of BLAAA is 10 ng/test. The total reaction volume is 210µl, wherein, specimen 10µl, R1 100µl, R2 100µl.
Figure 3 shows the calibration curves of tacrolimus LETIA with different dosages of BLAAA.
Figure 4 shows the functional sensitivity of latex enhanced competitive turbidimetric immunoassay to tacrolimus.
Figure 5A-Figure 5D show the comparison of tacrolimus concentrations in whole blood from 119 cases of patients determined by latex enhanced competitive turbidimetric immunoassay, ABBOTT CMIA, and LC-MS/MS. The solid line in the Bland-Altman diagram represents the average difference between two measurements, and the dashed line represents 95% CI of the average difference. Figure 5A and Figure 5C: Passing and Bablok regression curves; Figure 5B and Figure 5D: Bland-Altman deviation.

### Detailed description

Taking the detection of tacrolimus as an example, the implementation method of the present invention and its application effect are described below. The specific examples below are used to illustrate the present invention, but the scope of the present invention is not limited by the content of the examples. The experimental methods without specifying conditions in the following examples can be selected according to conventional methods and conditions, or according to the instructions of commercial kits.

Among the materials used in the following experiments, the carboxylated latex microsphere (PO323) and the blocking reagent Blockmaster DB1130 are from JSR (Japan). Anti-tacrolimus monoclonal antibody, tacrolimus-BSA complex and the calibrator were self-made (Feng-Bo Wu, Yun-Yun Yang, Xue-Bin Wang, Zhuo Wang, Wen-Wen Zhang, Zheng-Yue Liu, Yun-Qi Qian. A sample processing method for immunoassay of whole blood tacrolimus. Analytical Biochemistry. 2019, 576, 13-19). Tacrolimus precipitation reagent comes from Abbott Tacrolimus CMIA (Chemical Magnetic Immunoassay) kit. Biotin Quantitation Kit (Thermo-Fisher, USA) was used to determine the coupling rate of biotin-antibody. Other reagents were purchased from Sigma Company. All turbidimetric immunoassays were performed on Hitachi 7080 biochemical analyzer. The mass spectrometry detection system used is SCIEX Triple Quad 4500MD-Jasper^{™} (USA). ABBOTT Tacrolimus CMIA (reagent Lot number: 93006M800) was performed in Architect i1000. The coupling process of tacrolimus-latex was monitored by Nanotrac Wave II nanoparticle analyzer (USA). Hitachi CR21N high-speed freezing centrifuge (Japan) was used to wash latex and exchange buffers. ATS-e ultrasonic cell crusher for dispersing latex was purchased from ATS Company (China).

### Example 1 Tacrolimus LETIA based on detection mode A

### 1.1 Biotinylation of anti-tacrolimus antibodies

Biotin-labeled anti-tacrolimus antibody was prepared according to the method described in the literature (Feng-Bo Wu, Shi-Quan Han, Chao Zhang, and You-Feng He. Synthesis of a highly fluorescent β-diketone-europium chelate and its utility in Time-resolved fluoroimmunoassay of serum total thyroxin. Anal. Chem., 2002, 74, 5882-5889; Feng-Bo Wu, Hai-Qiao Ouyan, Xiao-Yan Tang and Zhen-Xian Zhou. Double-antigen sandwich time-resolved immunofluorometric assay for the detection of anti-hepatitis C virus total antibodies with improved specificity and sensitivity. Journal of Medical Microbiology. 2008, 57, 947-953), that is, the anti-tacrolimus monoclonal antibody was reacted with biotinylated-epsilon-aminocaproic acid-N-hydroxysuccinimide, and the free biotin reagent was removed by dialysis. The coupling rate of biotin to antibody was determined by HABA-Avidin method using biotin quantification kit (Thermo-Fisher), and biotin: antibody molecule =16:1.

### 1.2 Preparation of tacrolimus-coupled latex microspheres

50 mg of carboxylated latex microspheres were suspended in 5mL MES buffer (20 mM, comprising 2% NaCl, pH6.2), and 5 mg EDC and 50 mg N-hydroxysulfosuccinimide sodium (NHSS) were added, and the reaction was carried out at room temperature with stirring for 45 min. After centrifugation at 12000×g for 15min, the activated latex microspheres were ultrasonically resuspended in 5 mL of coupling buffer (100mM Hepes, pH7.5, comprising 2% NaCl), mixed with 250µl of physiological saline comprising 2.5mg tacrolimus-BSA, and incubated overnight with stirring at room temperature. Latex microspheres were centrifuged at 12000×g for 15min, resuspended in 10mL of washing pH buffer (20mM Hepes, pH8.5, comprising 0.5% NaCl, 0.05% Tween-20, 0.05% NaN₃), and ultrasonicated with a 3.5mm horn at the highest frequency of 35% for 30 seconds. Repeat the above steps of resuspension-washing-ultrasonication twice to ensure that free tacrolimus-BSA conjugates are removed. Finally, the microspheres were resuspended in pH buffer to obtain R2, and the absorbance of the prepared R2 was 2.6±0.6A at the wavelength of 546nm (UV-1900 Shimadzu ultraviolet-visible spectrophotometer).

### 1.3 Tacrolimus LETIA determination

Optimized tacrolimus LETIAreagent: R1 is 20mM MES (pH 6.1) buffer comprising 0.9% NaCl, 100±20 ng/mL biotin-labeled anti-tacrolimus antibody, 3mg/mL EDTA-2K, 3% PEG-8000, 2% sucrose and 0.5% BSA. R2 is 50mM Hepes (pH 7.5) buffer comprising 2% NaCl, 5% sucrose, 0.1% Tween-20, 0.5% Blockmaster DB1130, 8±2 µg/mL streptavidin (SA) and 0.1±0.05% tacrolimus-coupled latex microspheres.

Before the specimen is determined by LETIA, tacrolimus was extracted from EDTA whole blood by using precipitation reagent: 300µl of whole blood specimen or calibrator was transferred to 1.5mL EP tube with 300µl precipitation reagent, and after 10 seconds of strong vortex, it was centrifuged at 15,000×g for 5 minutes, then the supernatant of tacrolimus was poured into a specimen cup and transferred to a biochemical analyzer to determine tacrolimus.

Before testing, the equipment parameters were set according to the data in Table 1 below:

**Table 1 Equipment parameters of the biochemical analyzer**

| Wave length | 546nm/none (Main wavelength/sub wavelength) | Specimen | 10µl | Reaction method | Two point endpoint method |
|---|---|---|---|---|---|
| optical path of Colorimetric cup | 1cm | R1 | 100µl | Calibration type | Non-linear |
| reaction temperature | 37°C | R2 | 100µl | Reaction direction | Downward |

The test parameters are set according to the data in Table 2 below:

**Table 2 Formula of specimen to be tested**

| | formula of Calibrator determination | formula of Specimen determination |
|---|---|---|
| Calibrator | 10µl | - |
| Specimen | - | 10µl |
| R1 | 100µl | 100µl |
| The calibrator and specimen with the above dosage was mixed with R1 respectively, and incubated at 37°C for about 5 minutes to obtain mixed solution 1. | | |
| R2 | 100µl | 100µl |
| The mixed solution 1 and R2 were mixed well, and after incubation at 37°C for about 20 seconds, the absorbance value A1 was read; and after incubation for 5 minutes, the absorbance value A2 was read, and calculate ΔA=A₂-A₁ | | |

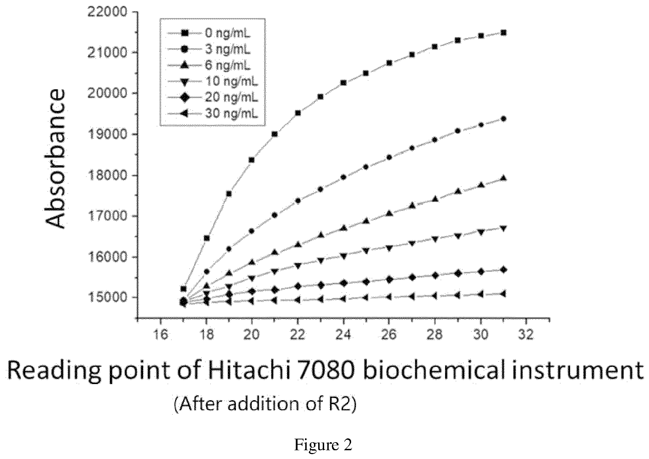

Figure 2 shows the agglutination progress curves of six calibrators when the dosage of BLAAA is 10 ng/test. The total reaction volume is 210µl, wherein, the calibrator 10µl, R1 100µl, R2 100µl.

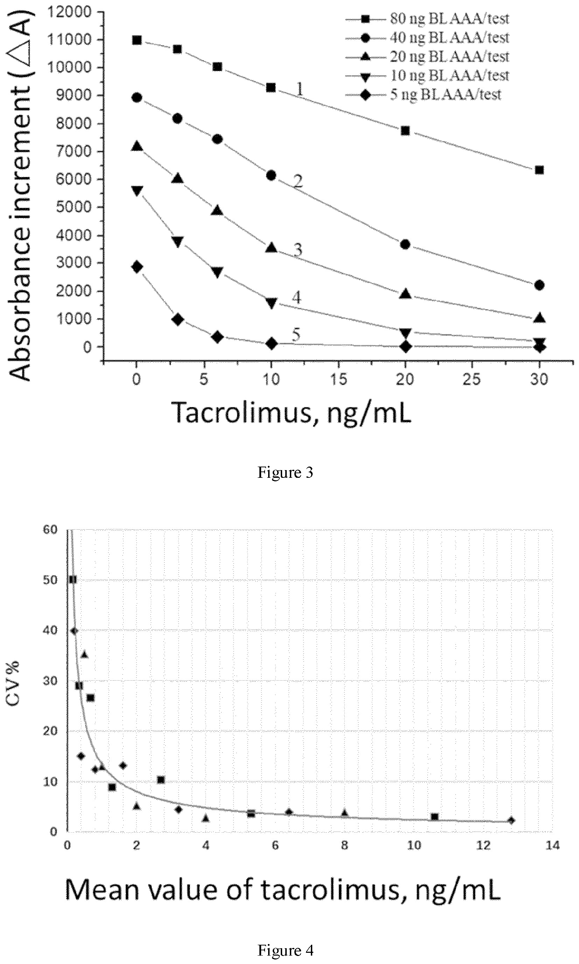

Figure 3 shows the calibration curves of tacrolimus LETIA with different dosages of BLAAA.

When the dosage of BLAAA is 5ng/test or 10ng/test, the LOD of tacrolimus LETIA is 0.12 ng/mL or 0.27 ng/mL, respectively, which is defined as the tacrolimus concentration on the calibration curve corresponding to the value obtained by subtracting 2SDs from the average ΔA of the calibrator with zero concentration for 24 times in parallel (Figure 3, curves 4 and 5).

### Example 2 Comparisons of clinical specimen determination values of tacrolimus LETIA, CMIA and LC-MS/MS based on detection mode A

Among the 119 cases of whole blood EDTA specimens used for comparative study, 81 cases were from renal transplant patients and 38 cases were from liver transplant patients. The concentrations of tacrolimus in these specimens were determined by LETIA, Abbott CMIA and LC-MS/MS as described in Example 1 respectively. Among them, ABBOTT Tacrolimus CMIA was performed in strict accordance with the kit instructions on the Architect I1000 analyzer, and LC-MS/MS is implemented as follows.

LC-MS/MS: SCIEX Triple Quad 4500MD-Jasper^{™} system (AB SCIEX) was used for LC-MS/MS analysis. Each 200µl of specimen and calibrator were added into a 2 ml EP tube, and then 1000µl acetonitrile comprising 5ng diazepam (internal standard) was added. The mixture is swirled for lmin and centrifuged at 13000×g for 8 minutes. 600µl of supernatant was taken into a 2mL EP tube, mixed with 600µl of pure water, vortexed for 10 seconds, and then centrifuged at 13,000×g for 8 minutes again. The supernatant was transferred to an automatic sampling bottle, and 10µl of supernatant specimen was injected into LC-MS/MS system. Tacrolimus was separated on Hypersil Gold C-18 column (3µm, 2.1x100mm), and the gradient mobile phase is consisted of solvent A (acetonitrile) and solvent B (pure water). The flow rate of all procedures is 0.4 mL/min. The column temperature is 45°C. The mobile phase is a mixture of 10% A and 90% B for the first 2 minutes, and then the solvent A is increased to 90% and kept for 3 minutes. Finally, the solvent A was reduced to 10% at 5.1 minute and kept for 2 minutes, then the next specimen was injected. Mass spectrometer settings were: spray voltage 5.5KV, curtain gas: 30psi, collision-activated dissociation (CAD): 9, source GS1: 45 psi, source GS2: 45 psi, drop temperature 400°C. The MRM m/z ratios monitored by tacrolimus and internal standard diazepam are m/z 821.5→768.5/786.6 and 285.0→7222.1, respectively. The TRIPLE QUADTM 4500MD system was used to integrate and quantify ion chromatographic peaks.

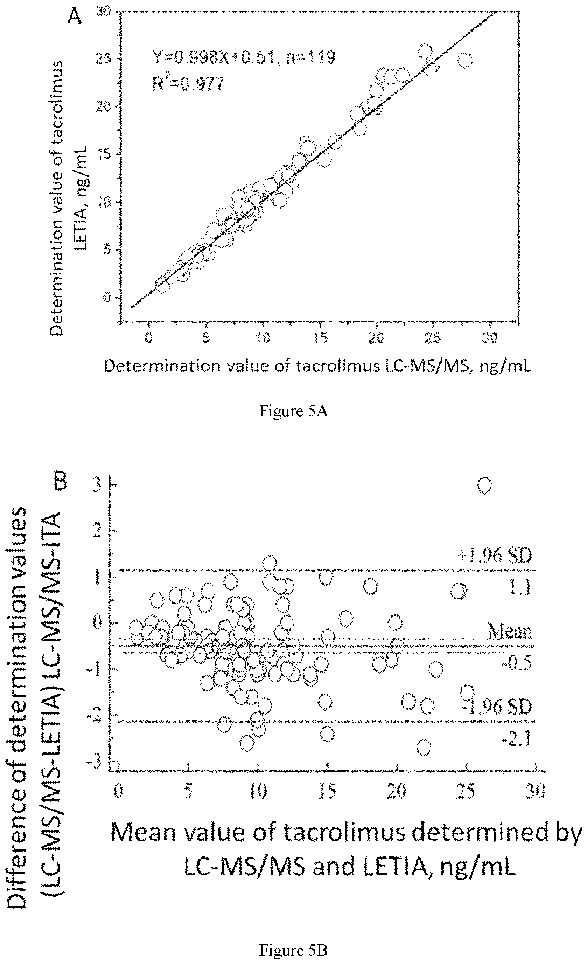

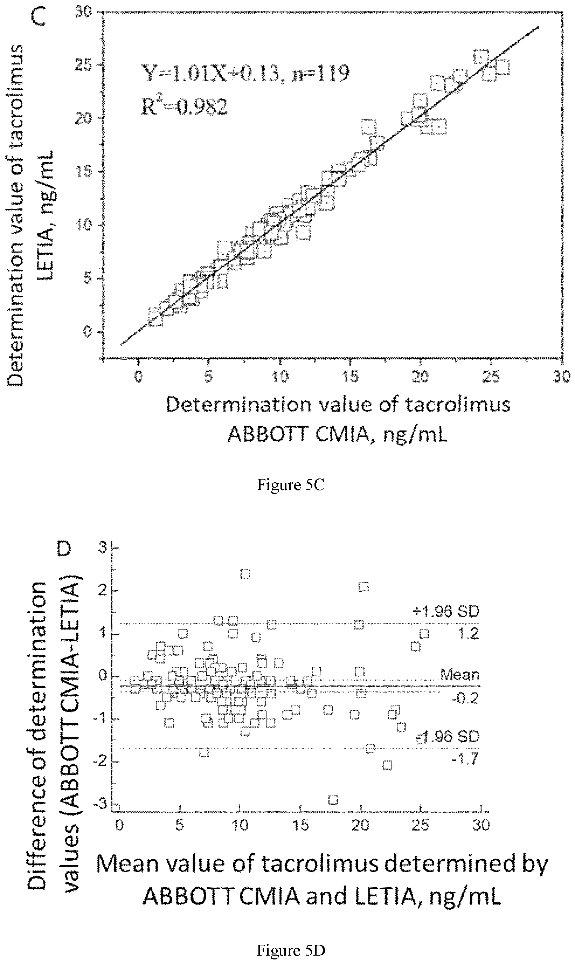

MedCalc-15.2.2 was used for linear regression and Bland-Altman analysis of concentrations of tacrolimus in specimen detected by LETIA, LC-MS/MS and ABBOTT CMIA (Figure 5). The linear regression equations are Y_{LETIA}=0.998X_{LC-MS/MS}+0.51 (R²=0.977) and Y_{LETIA}=1.01X_{CMIA}+0.13 (R²=0.982), and the average deviations of LETIA from CMIA and LC-MS/MS are -0.2ng/mL and -0.5ng/mL, respectively. Although the cross-reactivity of LETIA to tacrolimus metabolites was not detected, due to the high specificity of LC-MS/MS and the high consistency of measuring values between LETIA and LC-MS/MS, it can be inferred that the cross-reactivity of tacrolimus LETIA established in this example to tacrolimus metabolites does not affect the determination.

Figure 5 shows the consistency among LETIA, ABBOTT CMIA and LC-MS/MS methods for measuring the whole blood tacrolimus concentration of 119 patients. The solid line in the Bland-Altman diagram represents the average difference between two measurements, and the dashed line represents 95% CI of the average difference. A, C: Passing and Bablok regression curves; B, D: Bland-Altman deviation.

The following tacrolimus LETIA analysis performance evaluation experiments in Examples 3, 4 and 5 were carried out with reference to the method described in Example 1.

### Example 3 Functional sensitivity and precision of tacrolimus LETIA based on detection mode A

Three blood specimens with tacrolimus concentrations of 12.8ng/mL, 10.6ng/mL and 8ng/mL were doubled diluted to the concentration of tacrolimus smaller than 0.15ng/mL, and each specimen was repeatedly tested twice a day for consecutive 10 days, and the average concentration and %CV of each concentration were calculated. According to the obtained data, the average multiple curves fitting was used to fit (Figure 4). The functional sensitivity of tacrolimus is defined as the corresponding concentration of tacrolimus when the coefficient of variation (CV, %) is 20%, which is 0.59 ng/mL. Figure 4 shows the quantitative relationship between the every concentration of tacrolimus after doubled dilution in three blood specimens with the initial concentrations of tacrolimus in as 12.8ng/mL (diamond), 10.6ng/mL (square) and 8ng/mL(triangle), and its corresponding inter-experimental CV .

In the same determination, three whole blood specimens of tacrolimus with concentrations of 5.1, 13.9 and 20.2 ng/mL measured in parallel for 20 times, have the intra-experimental CV of 3.6%, 3.9% and 4.6% respectively. The intra-experimental CVs of the above three specimens were 3.3%, 5.2% and 5.1% respectively, after repeated determination for 20 days with an interval of 1 day.

Intra-experimental CV: the CV of the concentration value obtained by parallel porous determination of a specimen in the same determination.

Inter-experimental CV: the CV of the concentration value obtained by the determination of a specimen in different determination.

### Example 4 Tacrolimus LETIA recovery based on detection mode A

The tacrolimus specimen with high concentration (29.8ng/mL) was continuously diluted with the calibrator (zero-concentration calibrator) without tacrolimus, and the recovery rate was calculated according to the measured value and theoretical value. The results are shown in Table 3.

**Table 3 Dilution linearity of tacrolimus LETIA**

| Dilution factor | Measured concentration (ng/mL) | Calculated value (ng/mL)^{#} | Recovery rate (%)^{&} |
|---|---|---|---|
| Concentration before dilution | 29.8 | / | / |
| 1:1.1 | 27.9 | 30.7 | 103.0 |
| 1:1.3 | 21.6 | 28.1 | 94.3 |
| 1:1.5 | 19.1 | 28.7 | 96.3 |
| 1:1.7 | 18.2 | 30.9 | 103.7 |
| 1:2 | 14.6 | 29.2 | 98.0 |
| 1:4 | 7.5 | 30.0 | 100.7 |
| 1:10 | 2.8 | 28.0 | 94.0 |

| | | | |
|---|---|---|---|
| #: Calculated value = measured value multiplied by dilution factor &: Recovery rate (%) = (calculated concentration/concentration before dilution) ×100 | | | |

### Example 5 Anti-interference experiment of tacrolimus LETIA based on detection mode A

The following small molecules were used to test the cross-reaction of tacrolimus LETIA based on detection mode A in the present invention. Sirolimus and vermouth were added into the zero-concentration calibrator to a final concentration of both was 100 ng/mL. Cyclosporine was added into the zero-concentration calibrator to a concentration of 2000 ng/mL. Mycophenolic acid, amikacin, amphotericin B, amide, carbamazepine, chloramphenicol, furanilic acid, penicillin, kanamycin, vancomycin and teicoplanin were added into the zero-concentration calibrator to a concentrations all was 100µg/mL. The average concentration of tacrolimus (n=3) in all the above prepared specimens detected by tacrolimus LETIA was smaller than the functional sensitivity (0.59 ng/mL).

Tacrolimus was added to clinical specimens with cholesterol content of 339 mg/dL, triglyceride content of 727 mg/dL, and bilirubin content of 30.0 mg/dL to a concentration of 10ng/mL, and tacrolimus LETIA was used to detect the concentration of tacrolimus, and the error of the measured values was smaller than 15%.

As there is no commercialized tacrolimus metabolites, the degree of cross-reaction with tacrolimus metabolites was not detected. Interference from protein, such as rheumatoid factor (RF) and heterophile antibody, has not been detected, because such interfering substances have been precipitated and removed in the specimen pretreatment step before testing.

The above experimental results show that when the dosage of BLAAA is 5ng/ test and 10ng/ test, the tacrolimus LETIA established based on detection mode A can obtain an LOD of 0.12ng/mL and 0.27 ng/mL, respectively. The high consistency of specimen measurement values among tacrolimus LETIA, LC-MS/MS, and ABBOTT CMIA proves the measurement accuracy of the LETIA mode of the present invention. Based on these results, the latex enhanced competitive turbidimetric immunoassay method described in the present invention can not only be used to develop highly sensitive tacrolimus LETIA, but also be expected to become a universal method for the detection of other small molecules with low concentration. Based on the improvement of detection sensitivity, this method can expand the range of analyte to be detected in traditional LETIA technology.

## Claims

1. A method for latex enhanced competitive turbidimetric immunoassay, comprising the following steps:
(1) obtaining a mixed solution 1 by mixing and incubating a biotin labeled anti-analyte antibody (BLAAA) with a calibrator or specimen;
(2) mixing the mixed solution 1 in step (1) with avidin and a latex microsphere coupled with the analyte, and detecting initial absorbance A1, detecting absorbance A2 after incubation, and calculating absorbance increment ΔA, ΔA =A2-A1;
(3) establishing a calibration curve for the concentration of the analyte comprised in the calibrator by the absorbance increment ΔA of the calibrator, and realizing the quantitative detection of the concentration of the analyte in the specimen by comparison with the absorbance increment ΔA of the specimen;
or,
(1) obtaining mixed solution 1 by mixing and incubating a biotin labeled anti-analyte antibody (BLAAA) with a calibrator or specimen;
(2) obtaining mixed solution 2 by mixing and incubating the mixed solution 1 in step (1) with a latex microsphere coupled with the analyte;
(3) mixing the mixed solution 2 in step (2) with avidin, and detecting the initial absorbance A1, detecting the absorbance A2 after incubation, and calculating the absorbance increment ΔA, ΔA =A2-A 1;
(4) establishing a calibration curve for the concentration of the analyte comprised in the calibrator by the absorbance increment ΔA of the calibrator, and realizing the quantitative detection of the concentration of the analyte in the specimen by comparison with the absorbance increment ΔA of the specimen.

2. The method for latex enhanced competitive turbidimetric immunoassay according to claim 1, wherein the avidin is a group of proteins comprising four biotin binding sites and can bind to biotin with high affinity, and the avidin is avidin, avidin polymer, neutravidin, neutravidin polymer, streptavidin or streptavidin polymer.

3. The method for latex enhanced competitive turbidimetric immunoassay according to claim 1, wherein the analyte is a small molecule analyte; the small molecule analyte is digoxin, tacrolimus, cyclosporine, clozapine, aripiprazole, olanzapine, diazepam, quetiapine, risperidone, amitriptyline, duloxetine, nortriptyline, venlafaxine, citalopram, imipramine, sertraline, carbamazepine, oxcarbazepine, valproic acid, phenytoin, lamotrigine, levetiracetam, topiramate, aflatoxin, amphotericin b, voriconazole, posaconazole, itraconazole, teicoplanin, vancomycin, linezolid, amikacin, polymyxin b, tigecycline, chloramphenicol, melamine, fumonisin, aflatoxin M1, aflatoxin B1, clenbuterol or salbutamol.

4. The method for latex enhanced competitive turbidimetric immunoassay according to claim 1, wherein the molar ratio of the biotin to the anti-analyte antibody in the BLAAA is 5-25:1, preferably 10-20:1, and more preferably 12-17:1.

5. The method for latex enhanced competitive turbidimetric immunoassay according to claim 1, wherein the BLAAA, the avidin and the latex microsphere coupled with the analyte are stored and used in the form of pH buffer;
the concentration of the BLAAA is 1-1000 ng/mL, preferably 5-200 ng/ml;
the concentration of the avidin is 0.5-500 µg/mL, preferably 2-100 µg/ml;
the concentration of the latex microsphere coupled with the analyte is 0.01%-1%, preferably 0.05%-0.5%.

6. The method for latex enhanced competitive turbidimetric immunoassay according to claim 1, wherein the latex microsphere is a polystyrene latex microsphere with carboxyl on the surface; the average particle size of the polystyrene latex microsphere with carboxyl on the surface is preferably 50-500 nm, more preferably 100-400 nm; the carboxyl density on the surface of the polystyrene latex microsphere with carboxyl on the surface is preferably 0.02-0.40 mmol/g, more preferably 0.06-0.20 mmol/g.

7. A kit for latex enhanced competitive turbidimetric immunoassay, comprising:
(1) reagent 1 (R1), wherein the R1 is a pH buffer solution comprising BLAAA, and the BLAAA is a biotin labeled anti-analyte antibody;
(2) reagent 2(R2), wherein the R2 is a pH buffer solution comprising avidin and a latex microsphere coupled with the analyte;
(3) a calibrator, wherein the calibrator is a standard solution or a freeze-dried product comprising the analyte with a known concentration;
or,
(1) reagent 1 (R1), wherein the R1 is a pH buffer solution comprising BLAAA, the BLAAA is a biotin labeled anti-analyte antibody;
(2) reagent 2 (R2), wherein the R2 is a pH buffer solution comprising a latex microsphere coupled with the analyte;
(3) reagent 3 (R3), wherein the R3 is a pH buffer solution comprising avidin;
(4) a calibrator, wherein the calibrator is a standard solution or a freeze-dried product comprising the analyte with a known concentration.

8. The kit for latex enhanced competitive turbidimetric immunoassay according to claim 7, wherein the components of the kit for latex enhanced competitive turbidimetric immunoassay are as described in any one of claims 2 to 6.
